Europäisches Patentamt

**European Patent Office**

Office européen des brevets

(19)

(11) Veröffentlichungsnummer: **0 052 780**
A1

# (12) EUROPÄISCHE PATENTANMELDUNG

(21) Anmeldenummer: **81108945.7**

(22) Anmeldetag: **27.10.81**

(51) Int. Cl.³: **C 12 N 9/96**
**C 12 N 9/04, C 12 Q 1/32**

(30) Priorität: **26.11.80 DE 3044454**

(43) Veröffentlichungstag der Anmeldung:
**02.06.82 Patentblatt 82/22**

(84) Benannte Vertragsstaaten:
**AT BE CH DE FR GB IT LI LU NL SE**

(71) Anmelder: **BOEHRINGER MANNHEIM GMBH**
**Sandhofer Strasse 112-132 Postfach 31 01 20**
**D-6800 Mannheim 31-Waldhof(DE)**

(72) Erfinder: **Lessmann, Hans-Dieter, Dr. rer. nat.**
**Mörikestrasse 3 d**
**D-6945 Hirschberg-Grosssachsen(DE)**

(72) Erfinder: **Tüngler, Peter, Dr. rer. nat.**
**Lindenweg 18**
**D-3550 Marburg(DE)**

(72) Erfinder: **Heinemann, Helmut, Dr. rer. nat.**
**Kepplerstrasse 20**
**D-6900 Heidelberg(DE)**

(54) Stabilisierte Enzymzubereitung.

(57) Die Erfindung betrifft stabilisierte Enzymzubereitungen enthaltend Lactatdehydrogenase oder Malatdehydrogenase sowie verträgliche Füllstoffe und Stabilisatoren, dadurch gekennzeichnet, daß als Stabilisatoren Aspartat, α-Keto-glutarat oder Tartrat in der 1-250fachen Menge des Enzyms verwendet werden und Stabilisatoren und Füllstoffe zusammen die 10-1000fache Menge des Enzyms ausmachen, sowie Verfahren zu ihrer Herstellung.

EP 0 052 780 A1

Croydon Printing Company Ltd.

## Stabilisierte Enzymzubereitung

In der Biochemie und Lebensmittelanalytik spielt der Nachweis von Lactat bzw. Pyruvat mit Lactatdehydrogenase und der Nachweis von Malat- bzw. Oxalacetat mit Malatdehydrogenase eine wichtige Rolle. Die Enzyme lassen sich aus Muskelfleisch, insbesondere Herzmuskeln oder Hefe, isolieren und sind in mehr oder weniger hoch angereicherter Form von vielen Firmen im Handel. Da diese Enzyme in gereinigter Form eine hohe Aktivität besitzen, müssen sie zur Verarbeitung in Reagenzgemische mit geeigneten Füllstoffen verdünnt werden, um die Dosierung zu erleichtern. Die Verdünnung erfolgt dabei vorzugsweise durch Zusatz von Mannit, Milchzucker, Ammoniumsulfat oder ähnlichen leicht löslichen Substanzen, die die eigentliche enzymatische Reaktion nicht beeinflussen. Während die relativ hoch konzentrierten Enzyme relativ stabil sind, zeigt sich, daß durch die notwendige Verdünnung mit etwa dem tausendfachen an Mannit, die Stabilität der Enzyme stark abnimmt und bereits bei einer Lagerung von 3 Wochen bei 35°C nur noch 30-60 % der ursprünglichen Aktivität gefunden werden.

Es stellte sich deshalb die Aufgabe, geeignete Stabilisierungsmittel zu finden, mit denen sich solche verdünnten Enzyme ausreichend stabilisieren lassen, damit sie zu Reagenzgemischen verarbeitet werden können.

Übliche Enzymstabilisierungsmittel wie Albumin, Gelatine, Ammoniumsulfat oder Tenside erweisen sich als ungeeignet, da sie entweder in einer für die Testreaktion unzulässig hohen Konzentration angewendet werden müssen oder keine ausreichende Stabilisierung bewirken.

Überraschenderweise wurde gefunden, daß ein Zusatz von Aspartat, α-Ketoglutarat oder Tartrat die gewünschte Stabilisierung bewirkt, wobei vorzugsweise Dinatriumsalze eingesetzt werden, jedoch auch andere Salze, die die Enzymreaktion nicht stören, beispielsweise Kalium- oder Ammoniumsalze. Ein Zusatz des Dinatriumsalzes der Ethylendiamintetraessigsäure zur Pufferung und zur Komplexierung eventuell vorhandener Metallionen, hat sich weiterhin als vorteilhaft erwiesen. Das Stabilisierungsmittel wird in gleicher bis etwa 25o-facher Ge- wichtsmenge zugesetzt, 3- bis 1oo-fache Konzentrationen werden be- vorzugt.

Als Verdünnungsmittel wird vorzugsweise Mannit verwendet, wobei die Menge dieses Füllstoffes nicht kritisch ist. Aus praktischen Erwägungen dürfte ein Zusatz der 50- bis 1000-fachen Menge bevorzugt werden, da sich auf diese Art und Weise leicht handhabbare Enzymaktivitäten ergeben. Für den Fall, daß die Stabilisatoren in großer Menge angewendet werden, können diese gleichzeitig auch als Füllstoffe wirken, so daß die nicht stabilisierenden Füllstoffe in ihrer Menge entsprechend verringert oder weggelassen werden können. Die Stabilisatoren und Füllstoffe zusammen sollten etwa die 10-1000fache Menge des Enzyms ausmachen.

Wenn sich bei mechanischem Vermischen die trockenen Reagenzien nicht genügend homogen verteilen, um eine genaue Dosierung zu garantieren, erweist es sich als vorteilhaft, die Enzyme in einem geeigneten Lösungsmittel, beispielsweise Wasser, zu lösen und diese Lösung homogen auf das Verdünnungsmittel aufzubringen (Feuchtgranulation) bzw. das Verdünnungsmittel in einer größeren Menge der Enzymlösung zu lösen oder zu suspendieren und diese Mischung durch Sprühgranulation, Lyophilisation oder ähnliche Verfahren wieder zur Trockene zu bringen. Da größere Granulate sich häufig einfacher verarbeiten lassen als feine Pulver, werden die so erhaltenen Enzymverschnitte vorteilhaft feucht oder trocken zu Granulaten weiterverarbeitet und in dieser Form zur Herstellung der eigentlichen Reagenzmischungen benutzt.

Andere bi- bzw. polifunktionale Carbonsäuren wie Oxalsäure, Malonsäure, Adipinsäure, Schleimsäure, Pimelinsäure, Dodecandisäure, 3,3'-Thiodipropionsäure und Taurin erwiesen sich als wirkungslos. Da diese Verbindungen den erfindungsgemäßen Verbindungen im chemischen Aufbau weitgehend ähnlich sind, läßt sich nicht sagen, worauf die erfindungsgemäße Stabilisierung beruht.

In den folgenden Beispielen werden die erfindungsgemäßen stabilisierten Enzymmischungen näher erläutert und eine Reihe von nicht erfindungsgemäßen Mischungen gegenübergestellt:

- 4 -

Beispiel A (Vergleich)

785 mg (67 kU) LDH und 7,6 mg EDTA-Dinatriumsalz werden in 20 ml Wasser bei pH = 7,5 gelöst. Mit dieser Lösung werden 100,0 g Mannit granuliert. Das feuchte Granulat wird im Vakuum getrocknet.

Beispiel 1

640 mg (55 kU) LDH, 10,0 g Aspartat-Mononatriumsalz und 0,11 g EDTA-Dinatriumsalz werden in 100 ml Wasser bei pH = 7,5 gelöst und die Lösung lyophilisiert.

Beispiel 2

6,1 g (522 kU) LDH, 105,0 g Aspartat-Mono-Natriumsalz und 0,114 g EDTA-Dinatriumsalz werden in 275 ml Wasser bei pH = 7,5 aufgelöst und zusammen mit einer Lösung von 1 kg Mannit in 1,5 l Wasser sprühgetrocknet.

Beispiel 3

1,5 g (130 kU) LDH, 13,75 g α-Ketoglutarat-Dinatriumsalz und 10 mg EDTA-Dinatriumsalz werden in 36 ml Wasser bei pH = 7,5 gelöst. Mit dieser Lösung werden 250,0 g Mannit granuliert. Das feuchte Granulat wird im Vakuum getrocknet.

### Beispiel 4

0,6 g (52 kU) LDH und 5,25 g α-Ketoglutarat-Dinatriumsalz werden in 16,0 ml Wasser bei pH = 7,5 gelöst. Mit dieser Lösung werden 100,0 g Mannit granuliert. Das feuchte Granulat wird im Vakuum getrocknet.

### Beispiel 5

2,1 g (180 kU) LDH, 7,6 mg EDTA-Dinatriumsalz und 7,0 g Dinatriumtartrat werden in 15 ml Wasser bei pH = 7,5 gelöst. Mit dieser Lösung werden 100,0 g Dinatriumtartrat granuliert. Das feuchte Granulat wird im Vakuum getrocknet.

### Beispiel 6

0,55 g (47 kU) LDH und 5,25 g Dinatriumtartrat werden in 16 ml Wasser bei pH = 7,5 gelöst. Mit dieser Lösung werden 100,0 g Mannit granuliert. Das feuchte Granulat wird im Vakuum getrocknet.

### Beispiel B (Vergleich)

0,22 g (16 kU) MDH und 8 mg EDTA-Dinatriumsalz werden in 20 ml Wasser bei pH = 7,5 gelöst. Mit dieser Lösung werden 100,0 g Mannit granuliert. Das feuchte Granulat wird im Vakuum getrocknet.

### Beispiel 7

2,2 g (161 kU) MDH und 5,0 g Aspartat-Mononatriumsalz werden in 15 ml Wasser bei pH = 7,5 gelöst. Mit dieser Lösung werden 100,0 g Mononatriumaspartat granuliert. Das feuchte Granulat wird im Vakuum getrocknet.

Beispiel 8

2,2 g (161 kU) MDH, 75 mg EDTA-Dinatriumsalz und 5,0 g Aspartat-Mononatriumsalz werden in 15 ml Wasser bei pH = 7,5 gelöst. Mit dieser Lösung werden 100,0 g Aspartat-Mononatriumsalz granuliert. Das feuchte Granulat wird im Vakuum getrocknet.

Beispiel 9

0,18 g (13 kU) MDH, 10 g Aspartat-Mononatriumsalz und 0,11 g EDTA-Dinatriumsalz werden in 100 ml Wasser bei pH = 7,5 gelöst und die Lösung lyophilisiert.

Beispiel 10

3,4 g (256 kU) MDH und 3,3 g Tartrat-Dinatriumsalz werden in 9 ml Wasser bei pH = 7,5 gelöst. Mit dieser Lösung werden 50,0 g Tartrat-Dinatriumsalz granuliert. Das feuchte Granulat wird im Vakuum getrocknet.

Beispiel 11

3,4 g (256 kU) MDH und 3,3 g α-Ketoglutarat-Dinatriumsalz werden in 9 ml Wasser bei pH = 7,5 gelöst. Mit dieser Lösung werden 50 g α-Ketoglutarat-Dinatriumsalz granuliert. Das feuchte Granulat wird im Vakuum getrocknet.

## Tabelle 1

### Stabilität von LDH-Verschnitten

| | Verdünnungsmittel/ Stabilisierungsmittel | Aktivität Start | 3 Wo/35°C | Aktivitäts- verlust |
|---|---|---|---|---|
| | | U/mg | U/mg | % |
| Beispiel A (Vergleich) | Mannit EDTA-Na$_2$ | 0,52 | 0,25 | 51,9 |
| Beispiel 1 | Na-Aspartat EDTA-Na$_2$ | 4,18 | 4,328 | $\pm$ 0 |
| Beispiel 2 | Na-Aspartat EDTA-Na$_2$ Mannit | 0,52 | 0,482 | 7,3 |
| Beispiel 3 | α-Ketoglutarat-Na$_2$ EDTA-Na$_2$ Mannit | 0,48 | 0,39 | 18,8 |
| Beispiel 4 | α-Ketoglutarat-Na$_2$ Mannit | 0,36 | 0,27 | 25,0 |
| Beispiel 5 | Tartrat-Na$_2$ EDTA-Na$_2$ | 1,95 | 1,99 | $\pm$ 0 |
| Beispiel 6 | Tartrat-Na$_2$ Mannit | 0,37 | 0,36 | 2,7 |

Tabelle 2

Stabilität von MDH-Verschnitten

| | Verdünnungsmittel/ Stabilisierungsmittel | Aktivität | | Aktivitäts-verlust |
|---|---|---|---|---|
| | | Start | 3 Wo/35°C | |
| | | U/mg | U/mg | % |
| Beispiel B (Vergleich) | Mannit ... EDTA-Na$_2$ | 0,C36 | 0,023 | 36,1 |
| Beispiel 7 | Aspartat-Na | 1,43 | 1,36 | 4,9 |
| Beispiel 8 | Aspartat-Na$_2$ EDTA-Na$_2$ | 1,38 | 1,31 | 5,1 |
| Beispiel 9 | Aspartat-Na$_2$ EDTA-Na$_2$ | 1,93 | 2,07 | -7 |
| Beispiel 10 | Tartrat-Na$_2$ | 4,69 | 4,48 | 4,5 |
| Beispiel 11 | α-Ketoglutarat-Na$_2$ | 4,24 | 4,06 | 4,2 |

0052780

Patentansprüche

1. Stabilisierte Enzymzubereitung enthaltend Lactatdehydrogenase oder Malatdehydrogenase sowie verträgliche Füllstoffe und Stabilisatoren, dadurch gekennzeichnet, daß als Stabilisatoren Aspartat, α-Ketoglutarat oder Tartrat in der 1-250fachen Menge des Enzyms verwendet werden und Stabilisatoren und Füllstoffe zusammen die 10-1000fache Menge des Enzyms ausmachen.

2. Enzymzubereitung gemäß Anspruch 1, dadurch gekennzeichnet, daß bezogen auf einen Gewichtsteil Enzym 1 - 100 Teile Stabilisator enthalten sind.

3. Enzymzubereitung gemäß Anspruch 1 oder 2, dadurch gekennzeichnet, daß auf einen Gewichtsteil Enzym 100 - 1000 Gewichtsteile Füll- und Hilfsstoffe enthalten sind.

4. Enzymzubereitung nach einem der Ansprüche 1 bis 3, dadurch gekennzeichnet, daß ein neutraler oder schwach alkalischer Puffer enthalten ist.

5. Enzymzubereitung nach Anspruch 4, dadurch gekennzeichnet, daß der Puffer Ethylendiaminotetraessigsäuredinatriumsalz ist.

6. Verfahren zur Herstellung von stabilisierten Enzymzubereitungen gemäß einem der Ansprüche 1 bis 5, dadurch gekennzeichnet, daß das Enzym und die Stabilisatoren in einem geeigneten Lösungsmittel gelöst und die Lösung mit den Füll- und Hilfsstoffen homogen vermischt und getrocknet wird.

7. Verfahren nach Anspruch 6, dadurch gekennzeichnet, daß die Füll- und Hilfsstoffe mit der Enzymlösung granuliert werden.

8. Verfahren nach Anspruch 6, dadurch gekennzeichnet, daß die Füll- und Hilfsstoffe in dem Lösungsmittel gelöst oder suspendiert sind und die Trocknung durch Lyophilisation oder Sprühtrocknung erfolgt.

**Europäisches Patentamt**

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | <u>DE - B - 1 598 325</u> (CALBIOCHEM) | |
| | * Patentanspruch; Beispiel * | 1-8 |
| | -- | |
| X | <u>DE - B - 1 289 668</u> (BOEHRINGER) | |
| | * Spalte 1, Zeile 49 bis Spalte 2, Zeile 46 * | 1-3,6, 8 |
| | -- | |
| X | <u>GB - A- 1 153 109</u> (BOEHRINGER) | |
| | * Seite 1, Zeile 75 bis Seite 2, Zeile 43; Beispiele 1,2 und 6 * | 1-3, 6,8 |
| | -- | |
| X | <u>EP - A - 0 009 222</u> (AMERICAN <u>MONITOR</u>) | |
| | * Seiten 32 bis 35 * | 1 |
| | -- | |
| A | <u>US - A - 4 180 917</u> (E. CLIFFORD) | |
| | * Patentansprüche 1 und 2 * | 1 |
| | -- | |
| P,X | <u>EP - A - 0 034 213</u> (MONDROVICH) | |
| | * Beispiele V bis VIII * | 1 |
| | --------- | |

**KLASSIFIKATION DER ANMELDUNG (Int. Cl.³)**

C 12 N 9/96
9/04
C 12 Q 1/32

**RECHERCHIERTE SACHGEBIETE (Int. Cl.³)**

C 12 N 9/00
C 12 Q 1/00

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 17-03-1982 | DELANGHE |